# EUROPEAN PATENT APPLICATION

(11) **EP 0 994 103 A1**
(43) Date of publication of application: **19.04.2000**
(21) Application number: 98420186.3
(22) Date of filing: 13.10.1998
(51) Int. Cl.: C07C 253/14, C07C 255/50

(54) **Processes for preparing pesticidal intermediates**

(71) Applicant: Rhone Poulenc Agro, 69009 Lyon (FR)
(72) Inventor: Stephan, Dominique, 69330 Meyzieu (FR); Ratton, Patrick, 69780 Mions (FR); Viauvy, Agnès, 69700 Saint Andeol le Chateau (FR)

(57) **Abstract**

The invention relates to a process for the preparation of a compound of formula (I): 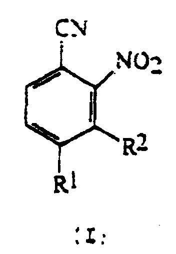 wherein:
R¹ represents C₁₋₄ haloalkyl, fluorine, chlorine or bromine; and R² represents hydrogen or C₁₋₄ alkoxy; which process comprises the reaction of the corresponding ortho-nitrochlorobenzene of formula (II): 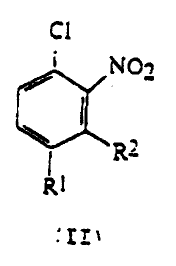
wherein R¹ and R² are as hereinbefore defined, with:
(a) cuprous cyanide and a source of bromide selected from hydrogen bromide, bromine and a tetraalkylammonium bromide; optionally in the presence of an alkali metal bromide or an alkaline earth metal bromide; or
(b) an alkali metal cyanide or a tetraalkylammonium cyanide, in the presence of cuprous bromide and a phase transfer catalyst; or
(c) cuprous cyanide and lithium iodide.

## Description

This invention relates to novel processes for preparing intermediates (particularly 4-cyano-3-nitrobenzotrifluoride) useful in the preparation of pesticides.

Pesticidal 4-benzoylisoxazoles, particularly 5-cyclopropylisoxazole herbicides and intermediate compounds in their synthesis, are described in the literature, for example in European Patent Publication Nos. 0418175, 0487353, 0527036, 0560482, 0609798 and 0682659.

Various methods for preparing these compounds are known. The present invention seeks to provide improved methods for the preparation of pesticides and the intermediate compounds useful in preparing them.

It is therefore an object of the present invention to provide novel processes for the preparation of ortho-nitrobenzonitrile compounds.

It is a further object of the present invention to provide processes for the preparation of ortho-nitrobenzonitrile compounds which proceed in high yield and/or with high selectivity.

The present invention allows these objects to be met in whole or in part.

### Summary of the Invention

The present invention accordingly provides a process for the preparation of an ortho-nitrobenzonitrile compound of formula (I): 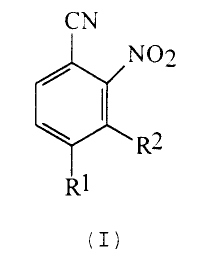 wherein:
R¹ represents C₁₋₄ haloalkyl (preferably trifluoromethyl) , fluorine, chlorine or bromine; and R² represents hydrogen or C_{1*-*4} alkoxy; which process comprises the reaction of the corresponding ortho-nitrochlorobenzene of formula (II): 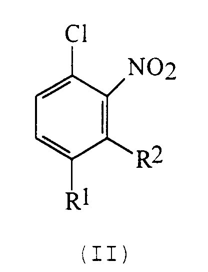 wherein R¹ and R² are as hereinbefore defined, with:
   (a) cuprous cyanide and a source of bromide selected from hydrogen bromide, bromine and a tetraalkylammonium bromide; optionally in the presence of an alkali metal bromide or an alkaline earth metal bromide; or
   (b) an alkali metal cyanide or a tetraalkylammonium cyanide, in the presence of cuprous bromide and a phase transfer catalyst; or
   (c) with cuprous cyanide and lithium iodide.

Certain compounds of formula (I) are known and a number of processes for their preparation and conversion into herbicidal 4-benzoylisoxazole derivatives have been described in the European Patent Applications cited above.

Compounds of formula (II) are known or may be prepared by known methods.

In formulae (I) and (II) and in the formulae depicted hereinafter, preferred values of the symbols are as follows:-

Preferably R¹ represents trifluoromethyl, fluorine or bromine; and R² represents hydrogen or methoxy.

In a particularly preferred embodiment of the invention R¹ represents trifluoromethyl and R² represents hydrogen.

It is to be understood that in this invention the term "alkyl" which forms part of tetralkylammonium salts represents a straight- or branched- chain alkyl group containing from one to six carbon atoms.

### Detailed Description of the Invention

The above preparation (a) of compounds of formula (I) from compounds of formula (II), is performed with cuprous cyanide and a source of bromide selected from hydrogen bromide, bromine and a tetraalkylammonium bromide, optionally in the presence of an alkali metal bromide or an alkaline earth metal bromide, preferably lithium bromide. In this process the amount of cuprous cyanide used is generally from 0.5-2 molar equivalents and preferably from 0.8-1.2 molar equivalents.

The amount of bromide source used is generally from 0.05-1 molar equivalent.

When an alkali metal bromide or an alkaline earth metal bromide is also present in the reaction mixture it is used in catalytic amount, generally from 0.01-0.5 molar equivalents and preferably from 0.02-0.05 molar equivalents.

The solvent may be chosen from nitriles such as acetonitrile or benzonitrile; ketones such as methyl isobutyl ketone; ethers such as tetrahydrofuran or diglyme (diethylene glycol dimethyl ether); esters such as methyl benzoate or n-butyl acetate; dimethylsulphoxide or sulpholane. Preferred solvents are acetonitrile, benzonitrile or diglyme.

The concentration of the compound of formula (II) used in the reaction solvent is generally in the range from 0.1ml/mmol to 2ml/mmol, and preferably from 0.2ml/mmol to 1ml/mmol.

The reaction temperature is generally from 100°C to 200°C, preferably from 130°C to 180°C

The above preparation (b) of compounds of formula (I) from compounds of formula (II), is performed with an alkali metal cyanide (for example sodium cyanide or potassium cyanide) or a tetraalkylammonium cyanide, in the presence of cuprous bromide and a phase transfer catalyst.

Preferably the alkali metal cyanide is potassium cyanide. The amount of alkali metal cyanide or tetraalkylammonium cyanide used is generally 1-1.5 molar equivalents (preferably 1-1.1 molar equivalents). The amount of cuprous bromide used is generally from 0.01-2 molar equivalents (preferably 1 molar equivalent). The reaction is conducted using solid/liquid phase transfer catalysis. The phase transfer catalyst may be selected from tetraalkylammonium salts or trialkylbenzylammonium salts (such as tetramethylammonium bromide or benzyltrimethylammonium bromide); phosphonium salts (such as tributylhexadecylphosphonium bromide); guanidinium salts (such as hexabutylguanidinium bromide or hexamethylguanidinium bromide); and crown ethers (such as 18-crown-6). The amount of phase transfer catalyst used is generally from 0.05-0.3 molar equivalents. Suitable solvents for the reaction include nitriles such as acetonitrile or benzonitrile; ethers such as tetrahydrofuran or diglyme (diethylene glycol dimethyl ether); ketones such as methyl isobutyl ketone; or esters such as methyl benzoate. The preferred solvent is acetonitrile.

The concentration of the compound of formula (II) used in the reaction solvent is generally in the range from 0.1ml/mmol to 2ml/mmol, and preferably from 0.2ml/mmol to 1ml/mmol.

The reaction temperature is generally from 100°C to 200°C, preferably from 130°C to 180°C.

The above preparation (c) of compounds of formula (I) from compounds of formula (II), is performed using cuprous cyanide and lithium iodide. Generally from 0.5-2 molar equivalents (preferably from 0.8-1.2 molar equivalents) of cuprous cyanide is employed in the reaction. The amount of lithium iodide employed is generally from 0.05 to 2 molar equivalents, preferably from 0.2 to 0.5 molar equivalents.

Suitable solvents for the reaction include nitriles such as benzonitrile or acetonitrile; ethers such as diglyme (diethylene glycol dimethyl ether); ketones such as methyl isobutyl ketone; or esters such as methyl benzoate.

The reaction temperature is generally from 100°C to 200°C, preferably from 130°C to 130°C.

The compounds of formula (I) obtained by the processes of the present invention may be used in the preparation of herbicidally active 4-benzoylisoxazole derivatives, for example according to the following reaction scheme:-

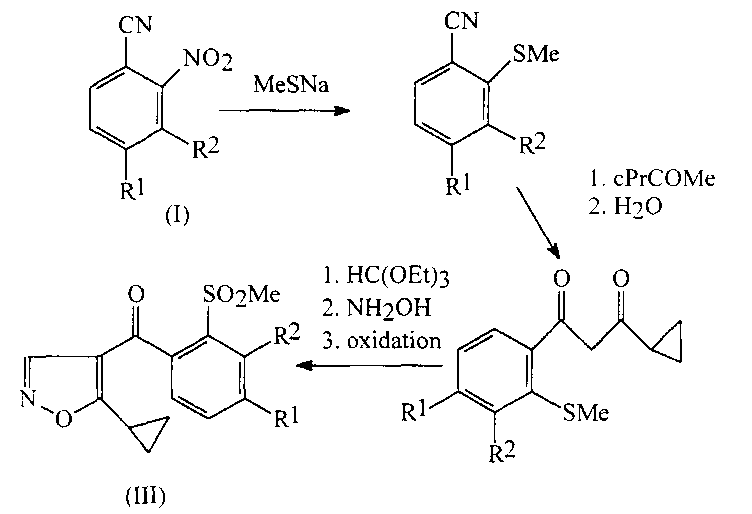

The 4-benzoylisoxazoles of formula (III) are described in for example European Patent Publication Nos. 0418175, 0527036 and 0560482

The following non-limiting examples illustrate the invention. Where concentrations of ingredients in solvent are given these are understood to refer to the concentration of the compound of formula (II) in the solvent (i.e. ml of solvent/mmol of compound of formula (II)).

### Example 1

### Preparation of 4-cyano-3-nitrobenzotrifluoride from 4-chloro-3-nitrobenzotrifluoride using cuprous cyanide and a source of bromide (process (a)):

A mixture of 4-chloro-3-nitrobenzotrifluoride (1 or 1.3 equivalents), cuprous cyanide (1 equivalent) and bromine (optionally in the presence of a catalytic amount of lithium bromide) or hydrogen bromide (47% aqueous) were mixed in benzonitrile (2 equivalents) at 20°C and heated at 170°C to give the title product. The results are shown in Table 1. The selectivity in these reactions was at least 90%.

**Table 1**

| **ClTNB(Eq)** | **Source of bromide** | | **Time** | **Conversion/ CuCN** |
|---|---|---|---|---|
| 1 eq | Br2 | 0.1 eq | 7h | 67% |
| 1.3eq | Br2 | 0.25 eq | 11h | 100% |
| | LiBr | 0.05 eq | | |
| 1eq | HBr aq(47%) | 0.5 eq | 8h | 62% |
| ClTNB = 4-chloro-3-nitrobenzotrifluoride. | | | | |

The above procedure was repeated using 4-chloro-3-nitrobenzotrifluoride (1 equivalent), cuprous cyanide (1 equivalent) and benzyltrimethylammonium bromide (1 equivalent) in acetonitrile (1ml/mmol) with heating at 160°C for 6 hours to give the title compound. The conversion of 4-chloro-3-nitrobenzotrifluoride was 94%, the yield of product 62% and the selectivity 66%.

The above experiment was repeated but with the following modifications; cuprous cyanide (1 equivalent) and tert-butylamine hydrobromide (1 equivalent) in benzonitrile (2 equivalents) were mixed at 20°C then heated to 150°C. 4-Chloro-3-nitrobenzotrifluoride (1.2 equivalents) was then added during 1 hour and the mixture maintained at that temperature for 7 hours. It may be seen that when these conditions are used, the reaction proceeds with good selectivity.

**Table 2**

| **tBuNH3.Br** | **Conversion/CuCN** | **Selectivity** |
|---|---|---|
| 1 eq | 87% | 90% |

### Example 2

### Preparation of 4-cyano-3-nitrobenzotrifluoride from 4-chloro-3-nitrobenzotrifluoride using an alkali metal cyanide, cuprous bromide and a phase transfer catalyst (process (b)):

A mixture of 4-chloro-3-nitrobenzotrifluoride (1 equivalent), potassium cyanide (1 equivalent), cuprous bromide (1 equivalent) and a quaternary ammonium salt (0.2 equivalents) was mixed at 20°C then heated with acetonitrile (1ml/mmol) at 160°C for 6 hours to give the title product. Table 3 shows the results from which it may be seen that good selectivity is obtained using these conditions.

**Table 3**

| **R4N.Br** | **Conversion(%)** | **Yield(%)** | **Selectivity(%)** |
|---|---|---|---|
| (a) | 80 | 64 | 80 |
| (b) | 79 | 66 | 84 |

| | | | |
|---|---|---|---|
| (a) tetraethylammonium bromide | | | |
| (b) benzyltrimethylammonium bromide | | | |

### Example 3

### Preparation of 4-cyano-3-nitrobenzotrifluoride from 4-chloro-3-nitrobenzotrifluoride using cuprous cyanide and lithium iodide (process (c)):

A mixture of 4-chloro-3-nitrobenzotrifluoride (1 equivalent), cuprous cyanide (1 equivalent) and lithium iodide (0.2 or 0.5 equivalents) was heated with benzonitrile or diglyme (diethylene glycol dimethyl ether) (1ml/mmol) for 6 hours at 160°C to give the title product. Lithium iodide was chosen for its solubility in organic solvents. Table 4 shows the results from which it may be observed that a particularly good selectivity is obtained using 0.5 equivalents of lithium iodide.

**Table 4**

| **Solvent** | **LiI** | **Conversion(%)** | **Yield(%)** | **Selectivity(%)** |
|---|---|---|---|---|
| PhCN | 0.5 eq | 62 | 62 | ≥95 |
| Diglyme | 0.5 eq | 70 | 70 | ≥95 |
| PhCN | 0.2 eq | 59 | 56 | 95 |
| Diglyme | 0.2 eq | 37 | 32 | 86 |

## Claims

1. A process for the preparation of a compound of formula (I): 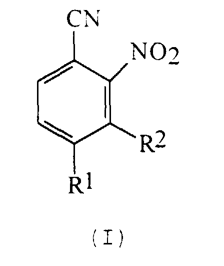 wherein:
R¹ represents C₁₋₄ haloalkyl, fluorine, chlorine or bromine; and R² represents hydrogen or C₁₋₄ alkoxy; which process comprises the reaction of the corresponding ortho-nitrochlorobenzene of formula (II): 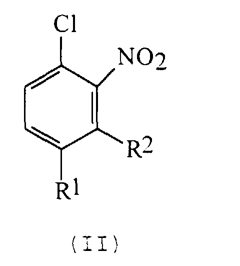 wherein R¹ and R² are as hereinbefore defined, with:
(a) cuprous cyanide and a source of bromide selected from hydrogen bromide, bromine and a tetraalkylammonium bromide; optionally in the presence of an alkali metal bromide or an alkaline earth metal bromide; or
(b) an alkali metal cyanide or a tetraalkylammonium cyanide, in the presence of cuprous bromide and a phase transfer catalyst; or (c) cuprous cyanide and lithium iodide.

2. A process according to claim 1 (a) in which 0.5-2 molar equivalents of cuprous cyanide is used.

3. A process according to claim 1 (a) or 2 in which 0.05-1 molar equivalents of bromide source is used.

4. A process according to claim 1 (a), 2 or 3 in which 0.01-0.5 molar equivalents of an alkali metal bromide or an alkaline earth metal bromide is used.

5. A process according to claim 1 (b) in which potassium cyanide is used.

6. A process according to claim 1 (b) or 5 in which 0.01-2 molar equivalents of cuprous bromide is used.

7. A process according to claim 1 (b), 5 or 6 in which the phase transfer catalyst is selected from tetraalkylammonium salts, trialkylbenzylammonium salts, phosphonium salts, guanidinium salts, and crown ethers.

8. A process according to any one of the preceding claims in which the concentration of the compound of formula (II) used in the reaction solvent is from 0.1ml,/mmol to 2ml/mmol.

9. A process according to claim 1 (c) in which 0.5-2 molar equivalents of cuprous cyanide is used.

10. A process according to claim 1 (c) or 9 in which the amount of lithium iodide used is from 0.05-2 molar equivalents.

11. A process according to any one of the preceding claims in which R¹ represents trifluoromethyl, fluorine or bromine; and R² represents hydrogen or methoxy.

12. A process according to any one of the preceding claims in which R¹ represents trifluoromethyl; and R² represents hydrogen.

13. A process according to claim 1 substantially as hereinbefore described.
